# EUROPÄISCHE PATENTSCHRIFT

(11) **EP 2 229 972 B1**
(45) Veröffentlichungstag und Bekanntmachung des Hinweises auf die Patenterteilung: **15.06.2016**
(21) Anmeldenummer: 10002729.1
(22) Anmeldetag: 16.03.2010
(51) Int. Cl.: A61M 16/04

(54) **Gesiebte Trachealkanüle**
Screened tracheal cannula
Sonde trachéale avec un tamis

(30) Priorität: 18.03.2009 DE 102009013424
(43) Veröffentlichungstag der Anmeldung: 22.09.2010
(73) Patentinhaber: Andreas Fahl Medizintechnik-Vertrieb GmbH, 51149 Köln (DE)
(72) Erfinder: Fahl, Andreas, 51107 Köln (DE)
(74) Vertreter: Geskes, Christoph

(56) Entgegenhaltungen:
- DE-A1- 10 109 935
- US-A- 2 301 338
- US-A- 5 207 655
- US-A1- 2002 078 962

## Beschreibung

Die Erfindung betrifft eine Trachealkanüle zur Einsetzung in ein Tracheostoma, wobei die Trachealkanüle zumindest ein Kanülenschild und zumindest ein dem Kanülenschild zugeordnetes Kanülenrohr umfasst, wobei das Kanülenrohr zumindest ein Sieb, eine distale Öffnung und eine mediale Öffnung umfasst.

Trachealkanülen sind aus dem Stand der Technik bekannt. In der Regel werden Trachealkaülen eingesetzt zur künstlichen Beatmung von Patienten mit Tracheostoma oder zur Atemunterstützung von spontan atmenden tracheotomierten oder laryngektomierten Patienten. Eine Trachealkanüle weist in der Regel eine Innenkanüle und eine Außenkanüle auf, wobei in einigen Ausgestaltungen bei sogenannten Sprechkanülen insbesondere für Patienten mit natürlichen oder künstlichen Stimmbändern die Innenkanüle und/oder die Außenkanüle gefenstert oder gesiebt ist. Insbesondere ist in einigen Ausgestaltungen die Innenkanüle gefenstert und die Außenkanüle gesiebt. Das Sieb soll ein Eindringen von Sekret in die Trachealkanüle und eine damit verbundene Respiration des Sekrets verhindern, des weiteren verhindert das Sieb eine Granulation im Bereich der Schleimhaut. Jedoch hat sich herausgestellt, dass auch durch die bekannten Siebe Sekret in die Trachealkanüle eindringt.

Aus der US 5,207,655 ist ein Endotrachealtubus bekannt, der kleinste als Ventilmittel dienende Öffnungen aufweist, welche beispielsweise bei Einspritzung eines Anästhetikums über einen Schlauch sich unter dem Druck des ausströmenden Anästhetikums öffnen und dieses abgeben.

Aus der US 2002/0078962 A1 ist eine Trachealkanüle gattungsgemäßer Art bekannt, die anstatt eines Siebes eine einzige Öffnung aufweist, die im gebogenen Teil der Trachealkanüle angeordnet ist.

Aus der DE 101 09 935 A1 schließlich ist eine Tracheostomaprothese gattungsgemäßer Art bekannt mit Perforierungen in Form eines Siebes in der Außenkanüle, welche jedoch nicht geeignet sind, das Eindringen von Sekret in die Trachealkanüle zu verhindern.

Aufgabe der Erfindung ist es eine verbesserte Trachealkanüle zur Verfügung zu stellen, welche die oben genannten Nachteile vermeidet.

Die Aufgabe wird erfindungsgemäß gelöst mittels einer Trachealkanüle nach Anspruch 1, einem Verfahren zum Abtransport von Sekret nach Anspruch 6 und einem Verfahren zur Herstellung einer Trachealkanüle nach Anspruch 10.

Es wird eine Trachealkanüle zur Einsetzung in ein Tracheostoma vorgeschlagen, wobei die Trachealkanüle zumindest ein Kanülenschild und zumindest ein dem Kanülenschild zugeordnetes Kanülenrohr umfasst, wobei das Kanülenrohr zumindest ein Sieb, eine distale Öffnung und eine mediale Öffnung umfasst. Die distale Öffnung definiert eine Einströmrichtung senkrecht zu einer distalen Öffnungsfläche. Das Sieb umfasst zumindest zwei Sieböffnungen, wobei die Sieböffnungen das Kanülenrohr in Einströmrichtung oder in einem Winkel zu einer senkrecht auf die Öffnungsfläche stehenden Horizontalebene durchstoßen, wobei der Winkel zur medialen Öffnung hin öffnet.

Unter Sieböffnung ist ein einzelner Durchtritt des Siebes durch das Kanülenrohr beziehungsweise ein einzelnes Loch in dem Material eines Kanülenrohrs zu verstehen. Somit weist die Sieböffnung zumindest jeweils eine Sieb-Öffnungsfläche auf der Innenseite und der Außenseite des Kanülenrohres sowie den materialfreien Raum dazwischen auf.

Unter einer Horizontalebene ist eine Ebene in der Ebene der Einströmrichtung zu verstehen, welche die Kanüle und vorzugsweise auch deren Öffnungsfläche schneidet und bei eingesetzter Kanüle horizontal, das heißt senkrecht zur Achse der Gravitationskraft beziehungsweise des Gravitationsvektors bei einem stehend oder aufrecht sitzenden Patienten ist. Insbesondere liegt ein Vektor der Einströmrichtung auf der Horizontalebene.

In einer Ausgestaltung ist die Öffnungsfläche zu einer senkrechten Ebene, auf der der Gravitationsvektor angeordnet ist, geneigt, vorzugsweise um bis zu ±10°. Beispielsweise ist die Öffnungsfläche um eine auf der Horizontalebene angeordnete Achse geneigt. In einer weiteren Ausgestaltung ist die Öffnungsfläche kippbar, vorzugsweise um bis zu ±10°. Beispielsweise wird eine Kippbarkeit der Öffnungsfläche durch ein bewegliches, am distalen Ende des Kanülenrohs angeordnetes Endstück bewirkt. Das Endstück umfasst in einer Ausgestaltung das Kanülenschild. Durch die Neigung oder Kippung der Öffnungsfläche wird insbesondere die Einströmrichtung beeinflusst.

Die vorgeschlagene Trachealkanüle hat den Vorteil, dass Sekret nicht mehr leicht in die Sieböffnungen eintreten kann. In einer Ausgestaltung durchstoßen die Sieböffnungen das Kanülenrohr in einem Winkel von etwa 0° bis 80° zur Horizontalebene. Insbesondere durchstoßen die Sieböffnungen das Kanülenrohr in einem Winkel von etwa 0° bis etwa 45° zur Horizontalebene. Weiterhin bevorzugt durchstoßen die Sieböffnungen das Kanülenrohr in einem Winkel von etwa 0° bis etwa 30°, weiterhin bevorzugt von etwa 0° bis etwa 15°. Auch sieht eine weiterhin bevorzugte Ausgestaltung vor, dass die Sieböffnungen die Trachealkanüle in einem Winkel von etwa 15° bis etwa 45°, insbesondere etwa 30° bis etwa 45°, weiterhin bevorzugt von etwa 45° bis 80° durchstoßen. Die angegebenen Winkel öffnen sich unterhalb der Horizontalebene. Vorzugsweise ist ein Winkelscheitel des Winkels auf einer Innenseite der Kanüle angeordnet. Weiterhin bevorzugt wird dadurch in einer Ausgestaltung bewirkt, dass die Sieböffnungen die Kanüle von der Innenseite zur Außenseite derart durchdringen, dass diese schräg von oben, ausgehend von der Innenseite, nach unten verlaufen.

In einer bevorzugten Ausgestaltung formen zumindest Teile eines Bodenbereiches oder einer Bodenfläche der Sieböffnung freiliegende Stufen. Dies hat den Vorteil, dass Sekret über die Stufen auf einer Außenseite der Trachealkanüle ablaufen kann, ohne das dieses in die Trachealkanüle eindringt. Insbesondere sind die Sieböffnungen in einer Schnittansicht senkrecht zur Horizontalebene des Kanülenrohrs terrassenförmig angeordnet.

Unter einem Bodenbereich beziehungsweise Bodenfläche ist ein Bereich der Sieböffungen zu verstehen, die sich in einer unteren Hälfte, vorzugsweise in einem unteren Drittel, bezogen auf die Horizontalebene der Sieböffnung, befindet. Insbesondere bildet die Sieböffnung einen Durchgang durch das Material, der einen runden oder ovalen Querschnitt aufweist beziehungsweise rohrförmig ist. In einer weiteren Ausgestaltung weist der Durchgang beziehungsweise die Sieböffnung einen quadratischen, rechteckigen, dreieckförmigen oder sonst wie gestalteten Querschnitt auf. Die einzelnen Sieböffnungen können auch unterschiedlich ausgestaltet sein. Insbesondere ist in einer Ausgestaltung vorgesehen, dass der Querschnitt über eine Längsrichtung respektive Durchstoßrichtung der Sieböffnung variiert, beispielsweise verjüngt sich die Sieböffnung zu einer Innenseite der Trachealkanüle und/oder einer Außenseite der Trachealkanüle hin.

Die Sieböffnung ist in einer Ausgestaltung zylindrisch ausgebildet, wobei unter zylindrisch zu verstehen ist, dass die Sieböffnung zwei Öffnungsflächen beliebiger Geometrie aufweist, die weitgehend gleich und zueinander verschoben sind. Insbesondere weist die Sieböffnung in einer Ausgestaltung eine schräg zylindrische Form auf.

Dass die Sieböffnungen das Kanülenrohr in Einströmrichtung oder in einem nach unten geöffneten Winkel zu einer senkrecht auf die Öffnungsfläche stehenden Horizontalebene durchstoßen, hat den Vorteil, dass eine Öffnungsfläche der Sieböffnungen größer ist als die Schnittfläche eines Durchmessers senkrecht zur Achse der Sieböffnung. Insbesondere ist die Öffnungsfläche der Sieböffnung vergrößert, wenn die Sieböffnungen auf einer Krümmung des Kanülenrohrs angeordnet sind, insbesondere wenn die von den Sieböffnungen durchstoßene Oberfläche des Kanülenrohrs gekrümmt ist. Durch die größere Öffnungsfläche im Vergleich zu aus dem Stand der Technik bekannten Sieböffnungen mit gleichem Durchmesser setzen sich die Sieböffnungen nicht so leicht mit Sekret zu.

In einer bevorzugten Ausgestaltung ist vorgesehen, dass die Trachealkanüle eine Innenkanüle und eine Außenkanüle umfasst. Insbesondere ist die Innenkanüle in einer Ausgestaltung gefenstert und die Außenkanüle gesiebt, oder umgekehrt. In einer weiteren Ausgestaltung ist die Innenkanüle und die Außenkanüle gesiebt. Weiterhin ist in einer Ausgestaltung vorgesehen, dass die Trachealkanüle weiterhin zumindest ein Sprechventil, eine künstliche Nase, ein Cuff und/oder eine Aufnahmevorrichtung umfasst. Insbesondere ist die Aufnahmevorrichtung für eine Aufnahme oder Befestigung von einem Beatmungsgerät - insbesondere einem mechanischen Ventilator oder einem Beatmungsbeutel, einem Inhalator, einer künstlichen Nase, einem Ventil, einem Schmuckstück oder einer sonstigen Vorrichtung vorgesehen. Auch kann über eine Aufnahmevorrichtung ein Teil, vorzugsweise ein Schlauch einer Absaugvorrichtung beispielsweise für Sekret eingeführt werden. In einer weiteren Ausgestaltung weist die Trachealkanüle, insbesondere die Außenkanüle eine Öffnung zum Absaugen von Sekret auf.

Besonders bevorzugt ist in einer Ausgestaltung vorgesehen, dass die Trachealkanüle zumindest ein Material aufweist aus einer Gruppe umfassend Silber, Silikon, Polyvinylchlorid, Polyethylen, Polystyrol, Polypropylen, Polycarbonat, Copolycarbonat und/oder Polyurethan.

Besonders bevorzugt ist in einer Ausgestaltung vorgesehen, dass als Material für die Kanäle ein Kunststoff ausgewählt ist aus einer Gruppe umfassend Silikon, Polyvinylchlorid, Polyethylen, Polystyrol, Polypropylen, Polycarbonat, Copolycarbonat und/oder Polyurethan. Insbesondere wird ein Kunststoff verwendet, der frei von Phthalaten ist, insbesondere frei von Diethylhexylphthalat. Weiterhin ist vorgesehen dass der Kunststoff insbesondere bei einer Verwendung von Polyvinylchlorid (C10-C21)-Alkansulfonsäurephenylester und/oder 1,2-Cyclohexan-dicarbonsäurediisononylester umfasst. Weiterhin ist eine Ausgestaltung vorgesehen, wobei ein Material verwendet wird, das Vinylacetat, Maleinsäure, Ethen, Vinylether und/oder Acrylsäuremethylester umfasst.

Mit der erfindungsgemäßen Trachealkanüle kann ein Verfahren zum Abtransport von Sekret durchgeführt werden, wobei die Trachealkanüle zumindest ein Kanülenschild und zumindest ein dem Kanülenschild zugeordnetes Kanülenrohr umfasst, wobei das Kanülenrohr zumindest ein Sieb, eine distale Öffnung und eine mediale Öffnung umfasst, wobei die distale Öffnung eine Einströmrichtung senkrecht zu einer distalen Öffnungsfläche definiert und das Sieb zumindest zwei Sieböffnungen umfasst. Zumindest Teile eines Bodenbereiches oder einer Bodenfläche der Sieböffnung formen freiliegende Stufen und Sekret wird über die Stufen der Sieböffnungen an einer Außenseite des Kanülenrohrs abgeleitet. Insbesondere wird das Sekret auf einer Außenseite einer Außenkanüle der Trachealkanüle abgeleitet.

Durch das beschriebene Verfahren kann eine Respiration des Sekretes weitgehend verhindert werden. Das Sekret, das sich an der Außenseite der Trachealkanüle ablagert, fließt insbesondere durch einen gravitativen Einfluss nach unten. Bei aus dem Stand der Technik bekannten Trachealkanülen sind die Sieböffnungen derart angeordnet, dass das Sekret bei der Abwärtsbewegung in die Sieböffnungen und somit in die Trachealkanüle eindringt. Hingegen wird bei dem beschriebenen Verfahren durch die stufenförmige Anordung der Sieböffnung ein Eindringen des Sekretes in die Trachealkanüle verhindet. Das Sekret läuft über die einzelnen Stufen ab, die in einer Schnittansicht orthogonal zur einer Horizontalebene terrassenförmig angeordnet sind. In einer Ausgestaltung ist vorgesehen, dass das Sekret bei verschlossener distaler Öffnung mittels eines Luftstroms, der durch die Sieböffnungen aus der Trachealkanüle heraus strömt, abgeleitet wird. Aus dem Stand der Technik ist bekannt, dass die Sieböffnungen bei verschlossener distaler Öffnung mittels eines Luftstroms, der durch die Sieböffnungen aus der Trachealkanüle heraus strömt, freigeblasen werden können. Dies hat jedoch den Nachteil, dass das Sekret sich wieder an der Außenseite der Trachealkanüle nahe der Sieböffnung ablagert, sodass ein Wiedereindringen des Sekretes in die Sieböffnung erfolgen kann. Hingegen wird bei dem beschriebenen Verfahren das Sekret mittels des Luftstromes in eine definierte Richtung bewegt, sodass eine Menge Sekret nicht zweimal in eine selbe Sieböffnung eindringen kann. Vielmehr wird eine Menge Sekret insbesondere durch den Luftstrom nach unten beziehungsweise medial geleitet. Insbesondere fließt die Menge Sekret stufenweise nach medial beziehungsweise unten ab. In einer Ausgestaltung ist vorgesehen, dass das Sekret zu einem an der Trachealkanüle angeordnetem Cuff abgeleitet wird. Beispielsweise kann das Sekret, dass sich im Bereich des Cuffs sammelt, abgesaugt werden. Ein Absaugen ist jedoch nicht in jedem Fall notwendig. Beispielsweise ist in einer Ausgestaltung vorgesehen, dass die Trachealkanüle kein Cuff aufweist.

Weiterhin betrifft die Erfindung ein Verfahren zur Herstellung eines Kanülenrohrs für eine erfindungsgemäße Trachealkanüle, wobei das Kanülenrohr zumindest ein Sieb, eine distale Öffnung und eine mediale Öffnung umfasst, wobei die distale Öffnung eine Einströmrichtung senkrecht zu einer distalen Öffnungsfläche definiert und das Sieb zumindest zwei Sieböffnungen umfasst. In einem Spritzgusswerkzeug wird zumindest ein Kern eingebracht und ein Kunststoff in das Spritzgusswerkzeug eingespritzt, sodass zumindest ein Kanülenrohr geformt wird, wobei zumindest ein Teil des Kerns zumindest die Sieböffnungen gestalten. In einer Ausgestaltung wird auch zumindest das Kanülenschild durch den Spritzgussvorgang geformt. In einer weiteren Ausgestaltung wird das Kanülenschild nachträglich an dem Kanülenrohr befestigt. In einer weiteren Ausgestaltung ist vorgesehen, dass das Kanülenschild einteilig mit dem Kanülenrohr verbunden ist.

Siebe in Trachealkanülen, die aus dem Stand der Technik bekannt sind, werden nachtäglich in das Kanülenrohr insbesondere durch Stanzen oder Bohren eingebracht. Das vorgeschlagene Verfahren hat den Vorteil, dass eine Nachbearbeitung des Kanülenrohres nicht notwendig ist. Des Weiteren ist vorteilhaft, dass durch die Verwendung von Kernen eine beliebige Durchstoßrichtung der Sieböffnungen durch das Kanülenrohr realisierbar ist. Weiterhin sind die geometrischen Gestaltungsmöglichkeiten der Sieböffnungen wesentlich vielseitiger als aus dem Stand der Technik bekannt. So sind beispielsweise nicht nur runde Querschnittsflächen der Sieböffnungen beziehungsweise grade zylindrische Sieböffnungen möglich, vielmehr können auch beliebige andere Formgebungen, wie beispielsweise rechteckige, dreieckige oder beliebig geformte Querschnittsflächen vorgesehen werden. Insbesondere ist durch Verwendung von Kernen auch eine Ausgestaltung möglich, bei der die Sieböffnung konisch ausgestaltet ist.

In einer Ausgestaltung ist vorgesehen, dass das Kanülenrohr Sieböffnungen aufweist, die in einem Schnitt insbesondere senkrecht zur Horizontalebene durch das Kanülenrohr terrassenförmig angeordnet sind. Die Sieböffnungen können an beliebigen Stellen am Kanülenrohr vorgesehen werden. Vorzugsweise sind die Sieböffnungen im Bereich einer Krümmung des Kanülenrohrs angeordnet. Insbesondere sind die Achsen der Sieböffnungen parallel zur Einströmrichtung angeordnet. In einer weiteren Ausgestaltung sind die Achsen der Sieböffnungen in einem beliebigen Winkel im Raum zur Einströmrichtung angeordnet, der unterhalb der Horizontalebene öffnet. Weiterhin ist in einer Weiterbildung vorgesehen, dass die Achsen der Sieböffnungen parallel zueinander verlaufen. Weiterhin ist vorgesehen, dass die Achsen der Sieböffnungen nicht parallel zueinander verlaufen In einer weiteren Ausgestaltung ist vorgesehen, dass einige Sieböffnungen parallel und einige Sieböffnungen nicht parallel zueinander verlaufen, insbesondere ist auch eine Kombination der oben beschriebenen Ausgestaltung vorgesehen. Auch ist in einer Ausgestaltung vorgesehen, dass die Sieböffnungen beliebig auf einem Umfang des Kanülenrohres angeordnet sind.

Weitere vorteilhafte Ausgestaltungen gehen aus den nachfolgenden Zeichnungen hervor. Die dort dargestellten Weiterbildungen sind jedoch nicht beschränkend auszulegen; vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf.

Es zeigen:
- Fig. 1: eine Trachealkanüle;
- Fig. 2: ein Teilschnitt einer Außenkanüle nach Fig. 1;
- Fig. 3: eine Schnittansicht eines Siebes in einer Außenkanüle nach Fig. 1;
- Fig. 4: eine weitere Schnittansicht eines Siebes in einem Kanülenrohr;
- Fig. 5: eine Innenkanüle nach Fig. 1; und
- Fig. 6: eine weitere Ausgestaltung einer Trachealkanüle.

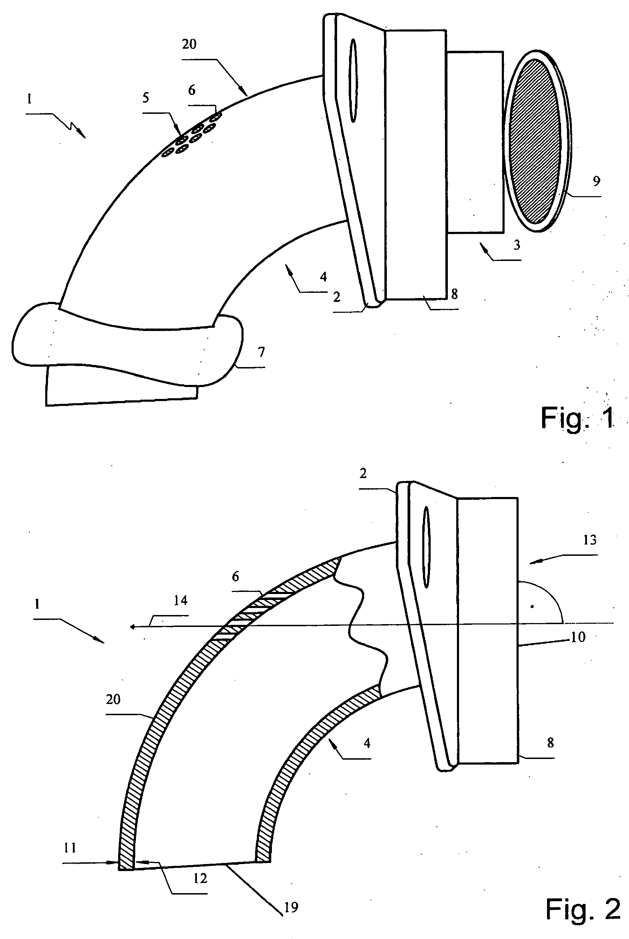

Fig. 1 zeigt eine Trachealkanüle 1 mit einem Kanülenschild 2, einer lnnenkanüle 3 und einer Außenkanüle 4. Die Innenkanüle 3 zumindest teilweise in der Außenkanüle 4 angeordnet. Das Kanülenschild 2 ist im Bereich des distalen Endes an der Außenkanüle 4 angeordnet. Die Außenkanüle weist ein Kanülenrohr 20 auf, wobei das Kanülenrohr 20 ein Sieb 5 mit Sieböffnungen 6 umfasst. Im Bereich eines medialen Endes des Kanülenrohres 20 ist in der gezeigten Ausgestaltung ein Cuff 7 angeordnet. In einer weiteren Ausgestaltung umfasst die Trachealkanüle 1 kein Cuff 7. Beispielsweise ist ein einer Variante vorgesehen, dass das Cuff 7 lösbar an der Trachealkanüle 1 angeordnet ist. Des weiteren weist die Tachealkanüle 1, insbesondere die Außenkanüle 4, eine Aufnahmevorrichtung 8 auf. Diese ist ausgestaltet zur Aufnahme, Fixierung respektive Befestigung der lnnenkanüle 3. In einer weiteren Ausgestaltung ist vorgesehen, dass die Aufnahmevorrichtung 8 oder die Innenkanüle 3 zur Aufnahme beispielsweise eines Beatmungsgeräts einer künstlichen Nase oder einem Sprechventil vorgesehen ist. Weiterhin ist in einer Ausgestaltung vorgesehen, dass die Aufnahmevorrichtung 8 an der Innenkanüle 3 beispielsweise zur Aufnahme von einer künstlichen Nase oder einem Sprechventil angeordnet ist. In der gezeigten Ausgestaltung ist ein Sprechventil 9 an der Innenkanüle 3 angeordnet. Dieses ist vorzugsweise lösbar und/oder öffbar an der Innenkanüle 3 angeordnet.

Fig. 2 zeigt einen Teilschnitt senkrecht zu einer hier nicht dargestellten Horizontalebene, auf welcher ein Vektor der Einströmrichtung 14 liegt und senkrecht aus der Bildebene ragt, einer Außenkanüle 4 durch das Kanülenrohr 20. Es ist zu erkennen, dass die Sieböffnungen 6 das Kanülenrohr 20 in Einströmrichtung 14 durchdringen. Die Sieböffnungen 6 haben die Funktion, bei geschlossener distalen Öffnung 10 einen Luftstrom, der sich aus Richtung der medialen Öffnung 19 hin zur distalen Öffnung 10 bewegt, aus der Trachealkanüle 1 zu leiten. Dies ermöglicht einem Halsatmer, der eine solche Trachealkanüle 1 trägt, die aus den Sieböffnungen 6 strömende Luft durch noch vorhandene natürliche oder künstliche Stimmbänder zu führen, um zu kommunizieren. Vorzugsweise wird die Trachealkanüle 1 mit einer Innenkanüle 3 verwendet, wie in Fig. 1 gezeigt. Die Innenkanüle 3 ist vorzugsweise gefenstert oder gesiebt, sodass die Luft aus der Innenkanüle 3 austreten und durch die Sieböffnungen 6 die Trachealkanüle verlassen kann.

Bei einem Einatmen wird die Luft senkrecht zur Öffnungsfläche 13 in Einströmrichtung 14 durch die distale Öffnung 10 in die Trachealkanüle 1 eingesogen und in Richtung mediale Öffnung 19 geführt. Bei einem Ausatmen wird die Luft ausgehend von der Trachea durch die mediale Öffnung 19 in die Trachealkanüle 1 geleitet. Insbesondere sorgt ein Cuff 7, wie in Fig. 1 dargestellt, für eine ausreichende Abdichtung, sodass die im wesentlichen gesamte Atemluft in die Trachealkanüle 1 eingeleitet wird. Ist die distale Öffnung 10 offen, so strömt die Atemluft aus der distalen Öffnung 10. Ist an der distalen Öffnung 10 beispielsweise ein Sprechventil 9 angeordnet, wird ein Druck innerhalb der Trachealkanüle 1 aufgebaut, sodass die Atemluft durch die Sieböffnungen 6 in die Trachea und an künstlichen oder natürlichen Stimmbändern vorbei in den Mundraum geleitet wird.

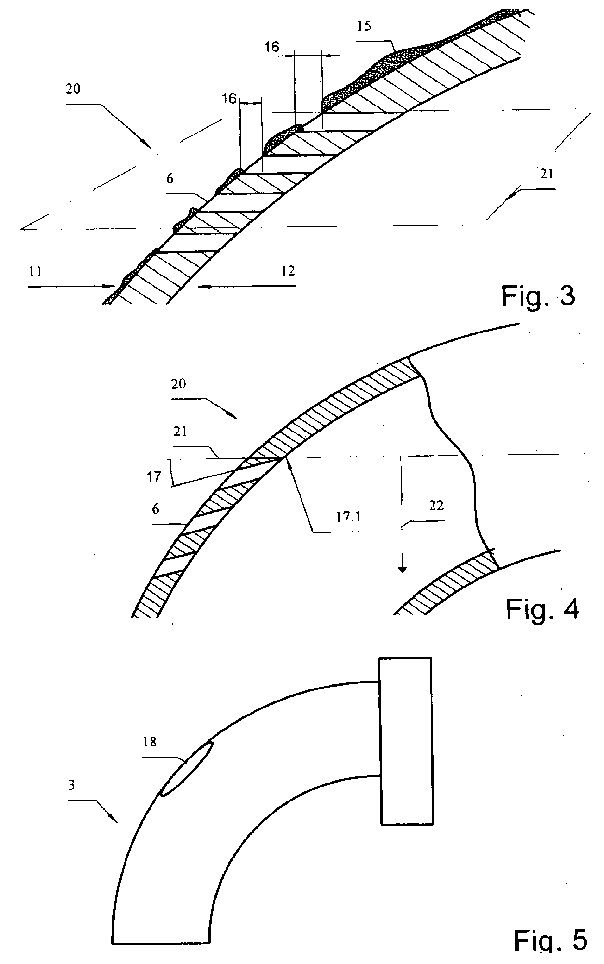

Fig. 3 zeigt eine Detailansicht eines Schnittes orthogonal zu einer Horizontalebene 21 durch das Kanülenrohr 20. Die Horizontalebene 21 ist als gestrichelte Fläche angedeutet und zur Verdeutlichung abweichend von der Schnittansicht perspektivisch nicht richtig dargestellt. In Fig. 3 sind die Sieböffnungen 6 zu erkennen. Auf dem Kanülenrohr 20 ist auf einer Außenseite 11 Sekret 15, insbesondere durch die Glandulae tracheales gebildetes seromuskolöses Sekret, abgesondert. Es ist zu erkennen, dass die Sieböffnungen 6 Stufen 16 bilden, die in der Schnittansicht gemäß Fig. 3 terrassenförmig angeordnet sind. Läuft das Sekret 15 über das Kanülenrohr 20, so dringt es nur teilweise in die Sieböffnungen 6 ein. Insbesondere wird durch die Anordnung der Sieböffnungen 6 ein Durchfließen der Sieböffnungen 6 mit Sekret 15 weitgehend verhindert, Sekret 15 ist somit auf der Innenseite 12 des Kanülenrohres 20 nicht oder kaum vorhanden. Vielmehr läuft das Sekret 15 über die durch die Sieböffnungen 6 geformten Stufen 16 nach unten ab.

Fig. 4 zeigt eine weitere Ausgestaltung des Kanülenrohres 20 beziehungsweise der Sieböffnungen 6. Die Sieböffnungen 6 durchstoßen das Kanülenrohr 20 in einem Winkel 17 zur Horizontalebene 21. Insbesondere steht die Horizontalebene 21 in Beziehung zu einem Gravitationsvektor 22, der senkrecht auf der Horizontalebene steht. Der Winkel 17 ist nach unten, das heißt in Richtung der in Fig. 2 dargestellten medialen Öffnung 19 geöffnet. Insbesondere ist Fig. 4 zu entnehmen, dass ein Winkelscheitel 17.1 des Winkels 17 auf der Innenseite des Kanülenrohrs 20 angeordnet ist. Durch die winklige Anordnung der Sieböffnungen wird Sekret, das in die Sieböffnungen geflossen ist, leichter und insbesondere auch ohne einen Luftstrom durch die Sieböffnungen 6 aus der Trachealkanüle aus der Sieböffnung 6 befördert.

Fig. 5 zeigt eine Innenkanüle 3 insbesondere zur Verwendung mit einer Außenkanüle 4, wie sie beispielsweise in Fig. 2 dargestellt ist. Die Innenkanüle 3 weist ein Fenster 18 auf, das vorzugsweise in einem montierten Zustand unterhalb eines Siebes einer Außenkanüle angeordnet ist. In weiteren Ausgestaltungen kann die Innenkanüle 3 ein Sieb aufweisen, das gemäß dem Stand der Technik ausgestaltet ist. Weiterhin kann vorgesehen sein, dass die Innenkanüle 3 ein erfindungsgemäßes Sieb aufweist.

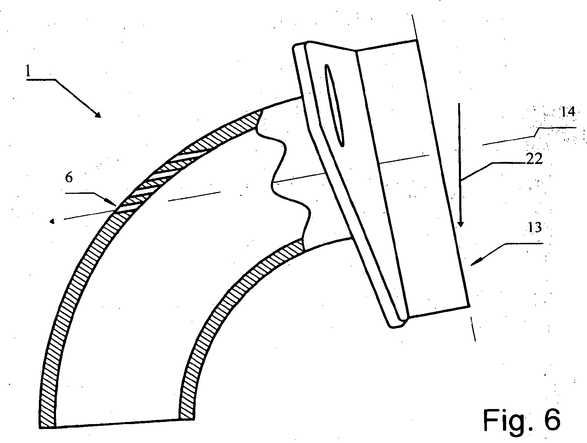

Fig. 6 zeigt einen weiteren Teilschnitt einer Trachealkanüle 1 mit einer zum Gravitationsvektor 22 geneigten Öffnungsfläche 13. Die Einströmrichtung 14, die senkrecht auf der Öffnungsfläche 13 steht, weist hier beispielhaft einen Winkel von etwa 10° zu einer hier nicht dargestellten Horizontalebene auf. Die Öffnungsfläche 13 ist somit zum Gravitationsvektor 22 derart geneigt, dass die Einströmrichtung 14 von oben distal nach unten medial gerichtet ist. Wie aus Fig. 6 deutlich zu entnehmen ist, sind die Sieböffnungen 6 in Einströmrichtung 14 in der Trachealkanüle 1 angeordnet. In einer weiteren Ausgestaltung ist vorgesehen, dass die Öffnungsfläche kippbar, das heißt beweglich ausgestaltet ist. Die Kippbarkeit wird insbesondere durch eine Beweglichkeit einzelner Teile der Trachealkanüle 1 ermöglicht. Die Einströmrichtung 14 ist somit variabel verstellbar. Die Sieböffnungen 6 oder einzelne Sieböffnungen 6 sind in dieser Ausgestaltung entlang einer der Graden einer Gradenschar der möglichen Einströmrichtungen 14 angeordnet. Beispielsweise kann die Beweglichkeit durch ein Zusammenwirken von Innenkanüle und Außenkanüle ermöglicht werden.

In einer hier nicht dargestellten Ausgestaltung der Trachealkanüle ist die Öffnungsfläche zum 13 Gravitationsvektor 22 derart gekippt, dass die Einströmrichtung 14 von unten distal nach oben medial gerichtet ist. Die in Einströmrichtung angeordneten Sieböffnungen 6 verlaufen dann durch die Kanüle von der Innenseite zur Außenseite derart, dass diese schräg von unten, ausgehend von der Innenseite, nach oben verlaufen. Ein Winkel von 10° sollte bei dieser Ausgestaltung jedoch nicht überschritten werden, da sonst Sekret in die Löcher eindringen könnte.

## Patentansprüche

1. Trachealkanüle (1) zur Einsetzung in ein Tracheostoma, wobei die Trachealkanüle (1) zumindest ein Kanülenschild (2) und zumindest ein dem Kanülenschild (2) zugeordnetes Kanülenrohr (20) umfasst, wobei das Kanülenrohr (20) zumindest ein Sieb (5), eine distale Öffnung (10) und eine mediale Öffnung (19) umfasst, wobei die distale Öffnung (10) eine Einströmrichtung (14) senkrecht zu einer distalen Öffnungsfläche (13) definiert und das Sieb (5) zumindest zwei Sieböffnungen (6) umfasst, **dadurch gekennzeichnet, dass** die Sieböffnungen (6) das Kanülenrohr (20) in Einströmrichtung (14) oder in einem Winkel (17) zu einer senkrecht auf die Öffnungsfläche (13) stehenden Horizontalebene (21) durchstoßen, wobei der Winkel (17) zur medialen Öffnung (19) hin öffnet.

2. Trachealkanüle (1) nach Anspruch 1, **dadurch gekennzeichnet, dass** die Sieböffnungen das Kanülenrohr (20) in einem Winkel (17) von 0° bis 80° zur Horizontalebene (21) durchstoßen.

3. Trachealkanüle (1) nach einem der vorhergehenden Ansprüche, **dadurch gekennzeichnet, dass** zumindest Teile eines Bodenbereiches oder einer Bodenfläche der Sieböffnung (6) freiliegende Stufen (16) formen.

4. Trachealkanüle (1) nach einem der vorhergehenden Ansprüche, **dadurch gekennzeichnet, dass** die Sieböffnungen (6) in einer Schnittansicht senkrecht zur Horizontalebene (21) des Kanülenrohrs (20) terrassenförmig angeordnet sind.

5. Trachealkanüle (1) nach einem der vorhergehenden Ansprüche, **dadurch gekennzeichnet, dass** die Trachealkanüle (1) weiterhin zumindest ein Sprechventil (9), eine künstliche Nase, ein Cuff (7) und/oder eine Aufnahmevorrichtung (8) umfasst.

6. Trachealkanüle (1) nach einem der vorhergehenden Ansprüche, **dadurch gekennzeichnet, dass** die Öffnungsfläche (13) zu einer senkrechten Ebene, auf der ein Gravitationsvektor (22) angeordnet ist, geneigt ist.

7. Verfahren zur Herstellung eines Kanülenrohrs (20) für eine Trachealkanüle nach einem der Ansprüche 1 bis 6, wobei das Kanülenrohr (20) zumindest ein Sieb (5), eine distale Öffnung (10) und eine mediale Öffnung (19) umfasst, wobei die distale Öffnung (10) eine Einströmrichtung (14) senkrecht zu einer distalen Öffnungsfläche (13) definiert und das Sieb (5) zumindest zwei Sieböffnungen (6) umfasst, **dadurch gekennzeichnet, dass** in einem Spritzgusswerkzeug zumindest ein Kern eingebracht wird und ein Kunststoff in das Spritzgusswerkzeug eingespritzt wird, sodass zumindest ein Kanülenrohr geformt wird, wobei zumindest ein Teil des Kerns zumindest die Sieböffnungen (6) gestalten.

8. Verfahren nach Anspruch 7, **dadurch gekennzeichnet, dass** der Kern derart in das Spritzgusswerkzeug eingebracht wird, dass das Kanülenrohr (20) Sieböffnungen (6) aufweist, die in einem Schnitt durch das Kanülenrohr (20) terrassenförmig angeordnet sind.

9. Verfahren nach Anspruch 7 oder 8, **dadurch gekennzeichnet, dass** der Kunststoff ausgewählt ist aus der Gruppe umfassend Silikon, Polyvinylchlorid, Polyethylen, Polystyrol, Polypropylen, Polycarbonat, Copolycarbonat und/oder Polyurethan.

## Claims

1. Tracheal cannula (1) for insertion into a tracheostoma, wherein the tracheal cannula (1) comprises at least one cannula plate (2) and at least one cannula tube (20) assigned to the cannula plate (2), wherein the cannula tube (20) comprises at least one sieve (5), one distal opening (10) and one medial opening (19), wherein the distal opening (10) defines a flow direction (14) perpendicular to a distal opening surface (13) and the sieve comprises at least two sieve openings (6), **characterized in that** the sieve openings (6) penetrate the cannula tube (20) in said flow direction (14) or at an angle (17) to a horizontal plane (21) perpendicular to the opening surface (13), wherein the angle (17) opens towards the medial opening (19).

2. Tracheal cannula (1) according to claim 1, **characterized in that** the sieve openings penetrate the cannula tube (20) at an angle (17) of 0° to 80° to the horizontal plane (21).

3. Tracheal cannula (1) according to anyone of the preceding claims, **characterized in that** at least parts of a bottom area or a bottom space of the sieve opening (6) form exposed steps (16).

4. Tracheal cannula (1) according to anyone of the preceding claims, **characterized in that** in a side view perpendicular to the horizontal plane (21) of the cannula tube (20) the sieve openings (6) are arranged in terraces.

5. Tracheal cannula (1) according to anyone of the preceding claims, **characterized in that** the tracheal cannula (1) further comprises at least one speaking valve (9), one artificial nose, one cuff (7) and/or one receptacle (8).

6. Tracheal cannula (1) according to anyone of the preceding claims, **characterized in that** the opening surface (13) is inclined towards a perpendicular plane, on which a gravitational vector (22) is arranged.

7. Method for producing a cannula tube (20) for a tracheal cannula according to one of the claims 1 to 6, wherein the cannula tube (20) comprises at least one sieve (5), one distal opening (10) and one medial opening (19), wherein the distal opening (10) defines a flow direction (14) perpendicular to a distal opening surface (13) and the sieve (5) comprises at least two sieve openings (6), **characterized in that** at least one core is introduced into an injection-molding tool and a plastic material is injected into the injection-molding tool, so that at least one cannula tube is formed, wherein at least one part of the core shapes at least said sieve openings (6).

8. Method according to claim 7, **characterized in that** the core is introduced into the injection-molding tool in a way that the cannula tube (20) shows sieve openings (6), which are in a cut through the cannula tube (20) arranged in terraces.

9. Method according to claim 7 or 8, **characterized in that** the plastic material is selected from the group comprising silicone, polyvinyl chloride, polythylene, polystyrene, polypropylene, polycarbonate, copolycarbonate and/or polyurethane.

## Revendications

1. Canule trachéale (1) à poser dans une trachéostomie, dans laquelle la canule trachéale (1) comprend au moins un bouclier de canule (2) et au moins un tube de canule (20) associé au bouclier de canule (2), dans laquelle le tube de canule (20) comprend au moins un tamis (5), une ouverture distale (10) et une ouverture médiale (19), dans laquelle l'ouverture distale (10) définit une direction d'inspiration (14) perpendiculaire à une face d'ouverture distale (13) et le tamis (5) comprend au moins deux ouvertures de tamis (6), **caractérisée en ce que** les ouvertures de tamis (6) percent le tube de canule (20) dans la direction d'inspiration (14) ou sous un angle (17) par rapport à un plan horizontal (21) perpendiculaire à la face d'ouverture (13), dans laquelle l'angle (17) s'ouvre vers l'ouverture médiale (19).

2. Canule trachéale (1) selon la revendication 1, **caractérisée en ce que** les ouvertures de tamis percent le tube de canule (20) sous un angle (17) de 0° à 80° par rapport au plan horizontal (21).

3. Canule trachéale (1) selon l'une quelconque des revendications précédentes, **caractérisée en ce qu'**au moins des parties d'une région de fond ou d'une face de fond de l'ouverture de tamis (6) forment des étages dégagés (16).

4. Canule trachéale (1) selon l'une quelconque des revendications précédentes, **caractérisée en ce que** les ouvertures de tamis (6), vues en coupe perpendiculairement au plan horizontal (21) du tube de canule (20), sont disposées en terrasses.

5. Canule trachéale (1) selon l'une quelconque des revendications précédentes, **caractérisée en ce que** la canule trachéale (1) comprend en outre au moins une soupape phonique (9), un nez artificiel, une manchette (7) et/ou un dispositif de réception (8).

6. Canule trachéale (1) selon l'une quelconque des revendications précédentes, **caractérisée en ce que** la face d'ouverture (13) est inclinée par rapport à un plan vertical, sur lequel un vecteur de gravitation (22) est appliqué.

7. Procédé de fabrication d'un tube de canule (20) pour une canule trachéale selon l'une quelconque des revendications 1 à 6, dans lequel le tube de canule (20) comprend au moins un tamis (5), une ouverture distale (10) et une ouverture médiale (19), dans lequel l'ouverture distale (10) définit une direction d'inspiration (14) perpendiculaire à une face d'ouverture distale (13) et le tamis (5) comprend au moins deux ouvertures de tamis (6), **caractérisé en ce que** l'on introduit au moins un noyau dans un outil de coulée par injection et on injecte une matière plastique dans l'outil de coulée par injection, de telle manière qu'au moins un tube de canule soitformé, dans lequel au moins une partie du noyau configure au moins les ouvertures de tamis (6).

8. Procédé selon la revendication 7, **caractérisé en ce que** l'on introduit le noyau dans l'outil de coulée par injection, de telle manière que le tube de canule (20) présente des ouvertures de tamis (6) qui, dans une coupe à travers le tube de canule (20), sont disposées en terrasses.

9. Procédé selon la revendication 7 ou 8, **caractérisé en ce que** la matière plastique est choisie dans le groupe comprenant la silicone, le chlorure de polyvinyle, le polyéthylène, le polystyrène, le polypropylène, le polycarbonate, le copolycarbonate et/ou le polyuréthane.
